# EUROPÄISCHE PATENTSCHRIFT

(11) **EP 1 272 505 B1**
(45) Veröffentlichungstag und Bekanntmachung des Hinweises auf die Patenterteilung: **24.08.2005**
(21) Anmeldenummer: 01940331.0
(22) Anmeldetag: 12.04.2001
(51) Int. Cl.: C07J 1/00, A61K 31/565, C07J 41/00, A61P 5/30

(54) **8BETA-SUBSTITUIERTE-11BETA-PENTYL-UND 11BETA-HEXYL-ESTRA-1,3,5(10)-TRIENDERIVATE**
8BETA-SUBSTITUTED-11BETA-PENTYL- AND 11BETA-HEXYL-ESTRA-1,3,5(10)-TRIENE DERIVATIVES
DERIVES DE 11BETA-PENTYLE- ET 11BETA-HEXYL-ESTRA-1,3,5(10)-TRIENE SUBSTITUES EN POSITION 8BETA

(30) Priorität: 12.04.2000 DE 10019167; 26.05.2000 US 207370 P
(43) Veröffentlichungstag der Anmeldung: 08.01.2003
(73) Patentinhaber: Schering Aktiengesellschaft, 13353 Berlin (DE)
(72) Erfinder: PETERS, Olaf, 07745 Jena (DE); BRAEUER, Nico, 07745 Jena (DE); HILLISCH, Alexander, 07743 Jena (DE); HEGELE-HARTUNG, Christa, 45470 Mülheim a.d. Ruhr (DE); FRITZEMEIER, Karl-Heinrich, 13505 Berlin (DE)
(86) Internationale Anmeldenummer: PCT/EP2001/004289
(87) Internationale Veröffentlichungsnummer: WO 2001/077138

(56) Entgegenhaltungen:
- WO-A-00/31112
- US-A- 3 681 407
- US-A- 3 736 345
- US-A- 3 806 546
- DATABASE CHEMABS [Online] CHEMICAL ABSTRACTS SERVICE, COLUMBUS, OHIO, US; NAGATA, WATARU ET AL: "8.beta.-Methylestradiols" retrieved from STN Database accession no. 73:25750 XP002175035 & JP 45 004059 B (SHIONOGI AND CO., LTD.) 10. Februar 1970 (1970-02-10)
- DATABASE CHEMABS [Online] CHEMICAL ABSTRACTS SERVICE, COLUMBUS, OHIO, US; NAGATA, WATARU ET AL: "8.beta.-Methylestranes" retrieved from STN Database accession no. 73:25749 XP002175036 & JP 45 004060 B (SHIONOGI AND CO., LTD.) 10. Februar 1970 (1970-02-10)
- DATABASE CHEMABS [Online] CHEMICAL ABSTRACTS SERVICE, COLUMBUS, OHIO, US; NAGATA, WATARU ET AL: "8.beta.-Methyl-9-dehydroestrone 3-ethers" retrieved from STN Database accession no. 73:25748 XP002175037 & JP 45 004061 B (SHIONOGI AND CO., LTD.) 10. Februar 1970 (1970-02-10)
- LOBACCARO C ET AL: "Steroidal Affinity Labels of the Estrogen Receptor. 3. Estradiol 11.beta.-n-Alkyl Derivatives Bearing a Terminal Electrophilic Group: Anti-estrogenic and Cytotoxic Properties" JOURNAL OF MEDICINAL CHEMISTRY, AMERICAN CHEMICAL SOCIETY. WASHINGTON, US, Bd. 40, Nr. 14, 4. Juli 1997 (1997-07-04), Seiten 2217-2227, XP002100729 ISSN: 0022-2623
- NAPOLITANO E ET AL: "11.beta.-Substituted Estradiol Derivatives. 2. Potential Carbon-11-and Iodine-Labeled Probes for the Estrogen Receptor" JOURNAL OF MEDICINAL CHEMISTRY, AMERICAN CHEMICAL SOCIETY. WASHINGTON, US, Bd. 38, Nr. 14, 7. Juli 1995 (1995-07-07), Seiten 2774-2779, XP002100730 ISSN: 0022-2623

## Beschreibung

### Feld der Erfindung

Die vorliegende Erfindung bezieht sich auf neue Verbindungen als pharmazeutische Wirkstoffe, die in vitro eine höhere Affinität an Estrogenrezeptorpräparationen von Rattenprostata als an Estrogenrezeptorpräparationen von Rattenuterus aufweisen und in vivo durch ihre präferentielle Wirkung am Ovar eine kontrazeptive Wirkung entfalten, deren Herstellung, ihre therapeutische Anwendung und pharmazeutischen Darreichungsformen, die die neuen Verbindungen enthalten.
Bei den chemischen Verbindungen handelt es sich um neuartige steroidale gewebeselektive Estrogene.

### Hintergrund der Erfindung

Kontrazeptive Methoden mit chemischen Verbindungen sind weit verbreitet bei Frauen, die nicht schwanger werden möchten. Folgende chemische Methoden der weiblichen Kontrazeption stehen uns derzeit zur Verfügung:
(A) Das endokrine Prinzip: Unterdrückung der Ovulation durch Hemmung der Gonadotropinfreisetzung und damit der Ovulation
(B) Verhinderung der Aszension von Spermien durch den weiblichen Reproduktionstrakt zum Eileiter wo die Befruchtung stattfindet
(C) Verhinderung der Implantation bzw. Nidation eines befruchteten Embryos in die Gebärmutter
(D) Spermicide
(E) Abortauslösende Mittel
Orale Kontrazeptiva, die aus unterschiedlichsten Kombinationen von einem Östrogen mit einem Gestagen bestehen, sind die am häufigsten angewandten Verhütungsmittel der Frau. Sie wirken nach dem endokrinen Prinzip. Obwohl derartige Verhütungsmittel sehr effektiv sind, so können doch unerwünschte Nebeneffekte auftreten wie z.B: irreguläre Blutungen, Übelkeit, Erbrechen, Depressionen, Gewichtszunahme oder Kopfschmerzen. Gelegentlich werden auch schwerere Erkrankungen beobachtet wie Thromboembolien, Schlaganfall, Leberadenome, Gallenblasenerkrankungen oder Hochdruck, die darauf hindeuten, daß heutzutage keine effektiven Kontrazeptiva ohne Nebenwirkungen verfügbar sind. Es existiert damit die medizinische Notwendigkeit nach einer neuen kontrazeptiven Methode.

Eine ideale kontrazeptive Methode ist eine Methode, die direkt am ovariellen Follikel ansetzt ohne die endokrine Hypothalamus-Hypophysen-Ovar Achse zu beeinflußen. Dies ist zu erreichen mit einer chemischen Verbindung, die die Follikulogenese beeinträchtigt, beispielsweise durch Zerstörung einer parakrinen Interaktion zwischen der Eizelle und den Granulosazellen, und damit dafür sorgt, daß
(a) das Follikelprogramm nicht adäquat ablaufen kann, so daß eine inkompetente Eizelle heranreift, die zwar ovuliert wird aber nicht befruchtet werden kann oder
(b) das Follikelprogramm nicht adäquat ablaufen kann, so daß eine inkompetente Eizelle heranreift, die zwar ovuliert und befruchtet wird, aber zu keiner Präimplantationsentwicklung führt oder
(c) die Follikulogenese nur eingeschränkt möglich ist und es zu keiner Ovulation kommt.

Follikelwachstum ist die Entwicklung eines ovariellen Follikels vom Primordialstadium bis hin zum großen antralen sprungreifen Follikel. Nur ein optimal aufgebauter antraler Follikel hat das Potential eine reife Eizelle zu ovulieren. Patientinnen mit ovarieller Infertilität, z.B. PCOS (=Polizystisches Ovar Syndrom) Patientinnen, haben eine gestörte Follikulogenese assoziiert mit Hormon- und Ovulationsstörungen sowie insuffizient gereifte Eizellen (Franks et al. (2000) Mol Cell Endocrinol 163: 49-52).

Es gibt immer mehr Hinweise dafür, daß die frühen Stadien der Follikulogenese, d.h. die Entwicklungsschritte vom Primordialfollikel bis hin zum frühen antralen Follikel, Gonadotropin-unabhängig sind, jedoch ist noch nicht abschließend geklärt welche der identifizierten autokrinen oder parakrinen Faktoren (Elvin et al. (1999), Mol Cell Endocrinol 13: 1035 -1048; McNatty et al. (1999), J Reprod Fertil Suppl 54: 3 - 16) die wichtigsten bei der frühen Follikulogenese sind. Gonadotropine, wie z. B. FSH (Follikel stimulierendes Hormon) dagegen sind hauptsächlich in die späten Schritte der Follikulogenese, d.h. der Entwicklung vom frühen antralen zum großen, ovulatorischen Follikel, involviert. Aber auch bei der späten Follikulogenese werden zusätzliche Modulatoren der Follikulogenese diskutiert (Elvin et al. (1999), Mol Cell Endocrinol 13: 1035 -1048).

Kürzlich wurde der Estrogenrezeptor-β (ERβ) als zweiter Subtyp des Estrogenrezeptors entdeckt (Kuiper et al. (1996), Proc. Natl. Acad. Sci. 93:5925-5930; Mosselman, Dijkema (1996) Febs Letters 392: 49-53; Tremblay et al. (1997), Molecular Endocrinology 11: 353-365). Das Expressionsmuster von ERβ unterscheidet sich von dem des ERα (Kuiper et al. (1996), Endocrinology 138: 863-870). Wogegen eine Expression von ERα in nahezu allen untersuchten Organen nachweisbar war, fand sich die höchste Expression von ERβ in weiblichen Tieren im Ovar, bei männlichen Tieren in der Prostata (Couse et al. (1997) Endocrinology 138: 4613-4621). Im Ovar zeigt sich eine deutliche ERβ Expression in Follikeln nahezu aller Entwicklungsstadien: Während in den Follikeln ERα nur in den äußeren Follikelzellen (Thekazellen) exprimiert wird, ist in den Östradiol-produzierenden Granulosazellen eine starke Expression von ERβ vorhanden. Aufgrund der verschiedenen Zellverteilung von ERα und ERβ im ovariellen Follikel ist damit zu rechnen, daß die Interaktion eines Liganden mit ERα bzw. ERβ zu unterschiedlichen zellulären Antworten führen wird. Daß ERα und ERβ funktionell unterschiedlich sind wurde kürzlich bestätigt durch die erfolgreiche Erzeugung von ERα und ERβ knockout Mäusen (Couse et al. (1999), Endocrine Reviews 20: 358-417). Demzufolge ist ERα maßgeblich beteiligt in der Funktion des Uterus, der Brustdrüse, der Steuerung der sexual-endokrinen Achse, wogegen ERβ überwiegend in die Vorgänge der ovariellen Physiologie einbezogen ist, insbesondere der Follikuolgenese und der Ovulation.

Ein weiteres Organsystem mit hoher ERβ-Expression ist der Testis (Mosselmann et al. 1996 Febs Lett 392 49-53) einschließlich der Spermatiden (Shugrue et al. 1998, Steroids 63: 498-504). Daß ERβ im männlichen Tier funktionell ist, ergibt sich auch durch Untersuchungen an ERα- (ERKO) bzw. ERβ-(βERKO)-Knockout-Mäusen: Männliche ERKO-Mäuse (Hess RA et al. 1997, Nature 390: 509-512) weisen deutliche Fertilitätsstörungen auf. Hierdurch wird die wichtige Funktion von Estrogenen hinsichtlich Aufrechterhaltung von Testisfunktion bezüglich der Fertilität belegt.

ERα und ERβ haben signifikant unterschiedliche Aminosäure-sequenzen in ihrer Liganden-bindungs- und Transaktivierungs-Domäne. Dies legt nahe, daß (1) ER Subtypen mit unterschiedlicher Affinität ihre Liganden binden und (2) daß Liganden unterschiedliches agonistisches und/oder antagonistisches Potential über die beiden Rezeptorsubtypen entfallten können.

Patentanmeldungen WO 00/47603, WO 00/63228, PCT/EP00/10804, DE 100 19167.3, US 60/207,370 sowie Publikationen (Sun et al. (1999), Endocrinology 140: 800-804; Stauffer et al. (2000), J Comb Chem 2: 318 - 329) zeigten kürzlich, daß steroidale und nichtsteroidale Liganden mit hoher Affinität an ERα und ERβ gefunden wurden. Einige Verbindungen waren beachtlich stärkere Agonisten/Antagonisten am ERα, wogegen andere Verbindungen stärkere Agonisten/Antagonisten am ERβ waren.

US-A-3,806,546 beschreibt 8β-methylestra-1,3,5(10)-trienen mit estrogenischer Aktivität aber erwähnt nichts gegenüber β-Estrogenrezeptor-Spezifizität.

In der WO 00/31112 werden neue steroidale Verbindungen basierend auf dem Grundkörper des, in 8-Position unsubstituierten, Estradiols beschrieben, die in 11β-Position einen Kohlenwasserstoffrest tragen, der eine einzelne lineare Kette mit einer Länge von 5 bis 9 Kohlenstoffatomen enthält. Diese Verbindungen haben ein ERα-agonistisches/ERβ-antagonistisches Wirkprofil. Aufgrund dieses gemischten Estrogenrezeptor-Profils sind diese Verbindungen als verbesserte Estrogene für die Behandlung von Estrogen-bedingten Störungen und zur Kontrazeption zusammen mit einem Gestagen geeignet.

In der US 60/271409 (nicht-vorveröffentlicht) werden zum ersten Mal in vivo Befunde gezeigt, aus denen deutlich wird, daß ERβ selective Agonisten zu einer Verbesserung der Follikulogenese führen, wogegen ERβ selektive Antagonisten die Fruchtbarkeit, d.h. die Ovulationsrate reduzieren.

Aufgabe der vorliegenden Erfindung ist es deshalb, Verbindungen bereitzustellen, die in vitro eine Dissoziation hinsichtlich Bindung an Estrogenrezeptorpräparationen von Rattenprostata und Rattenuterus aufweisen und in vivo durch ihre präferentielle Wirkung am Ovar eine kontrazeptive Wirkung entfalten ohne andere Östrogen-sensitiven Organe wie z.B. den Uterus oder die Leber zu beeinflußen. Ferner sollen diese Verbindungen verwendet werden zur Kontrazeption beim Mann sowie zur Behandlung von gutartigen oder bösartigen proliferativen Erkrankungen des Ovars.

Diese Aufgabe wird gelöst durch die Bereitstellung der Verbindungen der allgemeinen Formel I 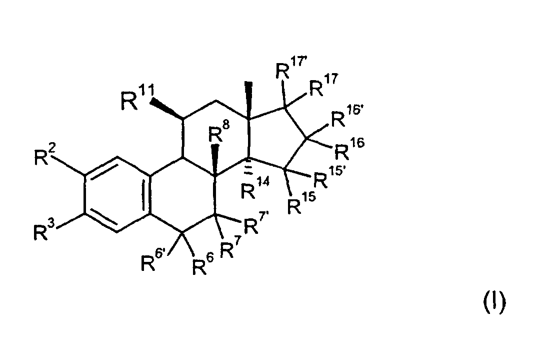 worin
- R²:: Wasserstoff, Halogen (F, Cl, Br, I);
einen Rest R¹⁸ oder R¹⁸O, wobei R¹⁸ Wasserstoff, ein Alkyl- oder Acylrest (beide gerad- oder verzweigtkettig, gesättigt oder ungesättigt mit bis zu 6 Kohlenstoffatomen), eine Trifluormethylgruppe;
einen Rest R¹⁹SO₂O, wobei R¹⁹ eine R²⁰R²¹N-Gruppe, worin R²⁰ und R²¹ unabhängig voneinander ein Wasserstoff, einen C₁-C₅-Alkylrest, eine Gruppe C(O)R²², worin R²² einen Kohlenwasserstoffrest (gegebenenfalls substituiert, gerad- oder verzweigtkettig, gesättigt oder bis zu dreifach ungesättigt, teilweise oder vollständig halogeniert) mit bis zu 10 Kohlenstoffatomen, einen gegebenenfalls substituierten C₃ - C₇ - Cycloalkylrest, einen gegebenenfalls substituierten C₄ - C₁₅ - Cycloalkyl alkyl rest oder einen gegebenenfalls substituierten Aryl-, Heteroaryl- oder Aralkylrest, oder, zusammen mit dem N-Atom, einen Polymethyleniminorest mit 4 bis 6 C-Atomen oder einen Morpholinorest);
- R³:: R¹⁸O, R¹⁹SO₂O, OC(O)R²², mit R¹⁸, R¹⁹, R²² in der unter R² angegebenen Bedeutung, sowie R¹⁸ zusätzlich einen Aryl-, Hetaryl- oder Aralkyl-Rest;
- R⁶, R^{6'}:: je ein Wasserstoff oder R⁶ eine zusätzliche Bindung mit R⁷;
- R⁷, R^{7'}:: je ein Wasserstoff oder R⁷ eine zusätzliche Bindung mit R⁶;
- R⁸:: einen Alkyl-, Alkenylrest (gerad- oder verzweigtkettig, teilweise oder vollständig halogeniert), jeweils mit bis zu 5 Kohlenstoffatomen, einen Ethinyl oder Prop-1-inylrest;
- R¹¹:: einen n-Pentyl- oder n-Hexylrest
- R¹⁴:: Wasserstoff oder eine zusätzliche Bindung mit R¹⁵;
- R¹⁵:: Wasserstoff oder eine zusätzliche Bindung mit R¹⁴ oder R¹⁶;
- R¹⁶:: Wasserstoff oder eine zusätzliche Bindung mit R¹⁵;
- R^{15'}, R^{16'}:: unabhängig voneinander Wasserstoff, Halogen, eine Gruppe R¹⁸O, R¹⁹SO₂O oder OC(O)R²² mit R¹⁸, R¹⁹, R²² in der unter R² angegebenen Bedeutung;
- R¹⁷, R^{17'}:: je ein Wasserstoffatom;
ein Wasserstoffatom und ein Halogenatom;
ein Wasserstoffatom und eine Benzyloxygruppe;
ein Wasserstoffatom und eine Gruppe R¹⁹SO₂-O-;
eine Gruppe R¹⁸ und eine Gruppe -C(O)R²² oder -O-C(O)R²²;
eine Gruppe R¹⁸-O- und eine Gruppe R¹⁸-;
eine Gruppe R¹⁸-O- und eine Gruppe -O-C(O)R²², mit R¹⁸, R¹⁹ und R²² in
der unter R² angegebenen Bedeutung oder
- R¹⁷, R^{17'}:: gemeinsam eine Gruppe =CR²³R²⁴, worin R²³ und R²⁴ unabhängig voneinander ein Wasserstoffatom und ein Halogenatom, oder gemeinsam ein Sauerstoffatom darstellen;
bedeuten.

Die möglichen Substituenten an den Kohlenstoffatomen 6, 7, 15, 16 und 17 können jeweils in der α- oder β-Position stehen.

In den Verbindungen der allgemeinen Formel I sowie in den beanspruchten Teilstrukturen kann für ein Halogenatom immer ein Fluor-, Chlor-, Brom- oder lodatom stehen; ein Fluoratom ist jeweils bevorzugt.
Insbesondere handelt es sich bei den Kohlenwasserstoffresten, die teilweise oder vollständig halogeniert sein können, um fluorierte Reste.

Der Kohlenwasserstoffrest R¹⁸ ist beispielsweise ein Methyl, Ethyl, Propyl, Isopropyl, Butyl, Isobutyl, tert.-Butyl, Pentyl, lsopentyl, Neopentyl, Heptyl, Hexylrest.

Die Alkoxygruppe OR¹⁸ kann 1 bis 6 Kohlenstoffatome enthalten, wobei Methoxy-, Ethoxy- Propoxy- lsopropoxy- und t-Butyloxygruppen bevorzugt sind.

Vertreter für die C₁-C₅-Alkylreste R²⁰ und R²¹ sind Methyl, Ethyl, Propyl, Isopropyl, Butyl, Isobutyl, tert.-Butyl, Pentyl, Isopentyl, Neopentyl.

Als Vertreter für gerad- oder verzweigtkettige Kohlenwasserstoffreste R²² mit 1 bis max. 10 Kohlenstoffatomen sind beispielsweise Methyl, Ethyl, Propyl, Isopropyl, Butyl, Isobutyl, tert.-Butyl, Pentyl, Isopentyl, Neopentyl, Heptyl, Hexyl und Decyl zu nennen; Methyl, Ethyl, Propyl und Isopropyl sind bevorzugt.

Als perfluorierte Alkylgruppen seien beispielsweise Trifluormethyl, Pentafluorethyl und Nonafluorbutyl genannt. Vertreter der teilweise fluorierten Alkylgruppen sind zum Beispiel 2,2,2-Trifluorethyl, 5,5,5,4,4-Pentafluorpentyl, 6,6,6,5,5,4,4,3,3-Nonafluorhexyl etc..

Als C₃-C₇-Cycloalkylgruppe ist eine Cyclopropyl-, butyl-, pentyl-, hexyl- oder heptylgruppe zu nennen

Ein C₄-C₁₅-Cycloalkylalkylrest weist 3 bis 7 Kohlenstoffatome im Cycloalkylteil auf; typische Vertreter sind die direkt vorstehend genannten Cycloalkylgruppen. Der Alkylteil weist bis zu 8 Kohlenstoffatome auf.
Als Beispiele für einen C₄-C₁₅-Cycloalkylalkylrest seien die Cyclopropylmethyl-, Cyclopropylethyl-, Cyclopentylmethyl-, Cyclopentylpropylgruppe etc. genannt.

Beim einem Arylrest handelt es sich im Sinne der vorliegenden Erfindung um einen Phenyl-, 1- oder 2-Naphthylrest; der Phenylrest ist bevorzugt.

Beispiele für einen Heteroarylrest sind der 2-, 3- oder 4-Pyridinyl-, der 2- oder 3-Furyl-, der 2- oder 3-Thienyl-, der 2- oder 3-Pyrrolyl, der 2-, 4- oder 5-Imidazolyl-, der Pyrazinyl-, der 2-, 4- oder 5-Pyrimidinyl- oder 3- oder 4-Pyridazinylrest.

Als Substituenten für einen Aryl- oder Heteroarylrest seien zum Beispiel ein Methyl-, Ethyl-, Trifluormethyl- Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, erwähnt.

Bei einem Aralkylrest handelt es sich um einen Rest, der im Ring bis 14, bevorzugt 6 bis 10, C-Atome und in der Alkylkette 1 bis 8, bevorzugt 1 bis 4, C-Atome enthält. So kommen als Aralkylreste beispielsweise in Betracht Benzyl, Phenylethyl, Naphthylmethyl, Naphthylethyl, Furylmethyl, Thienylethyl, Pyridylpropyl. Die Ringe können einfach oder mehrfach substituiert sein durch Halogen, OH, O-Alkyl, CO₂H, CO₂-Alkyl, -NO₂, -N₃, -CN, C₁-C₂₀-Alkyl, C₁-C₂₀-Acyl, C₁-C₂₀-Acyloxy-Gruppen.

Die Alkylgruppen bzw. Kohlenwasserstoffreste können teilweise oder vollständig fluoriert oder substituiert sein durch 1-5 Halogenatome, Hydroxygruppen oder C₁-C₄-Alkoxygruppen.

Mit einem C₂-C₅-Alkenylrest ist in erster Linie ein Vinyl- oder Allylrest gemeint.

Eine oder mehrere Hydroxylgruppen an den C-Atomen 3, 16 und 17 können mit einer aliphatischen, gerad- oder verzweigtkettigen, gesättigten oder ungesättigten C₁-C₁₄-Mono- oder Polycarbonsäure oder einer aromatischen Carbonsäure oder mit einer α- oder β-Aminosäure verestert sein.
Als derartige Carbonsäuren zur Veresterung kommen beispielsweise in Betracht: Monocarbonsäuren: Ameisensäure, Essigsäure, Propionsäure, Buttersäure, Isobuttersäure, Valeriansäure, Isovaleriansäure, Pivalinsäure, Laurinsäure, Myristinsäure, Acrylsäure, Propiolsäure, Methacrylsäure, Crotonsäure, Isocrotonsäure, Ölsäure, Elaidinsäure.
Die Veresterung mit Essigsäure, Valeriansäure oder Pivalinsäure ist bevorzugt.
Dicarbonsäuren: Oxalsäure, Malonsäure, Bernsteinsäure, Glutarsäure, Adipinsäure, Pimelinsäüre, Korksäure, Azelainsäure, Sebacinsäure, Maleinsäure, Fumarsäure, Muconsäure, Citraconsäure, Mesaconsäure.
Aromatische Carbonsäuren: Benzoesäure, Phthalsäure, Isophthalsäure, Terephthalsäure, Naphthoesäure, o-, m- und p-Toluylsäure, Hydratropasäure, Atropasäure, Zimtsäure, Nicotinsäure, Isonicotinsäure.
Die Veresterung mit Benzoesäure ist bevorzugt.
Als Aminosäuren kommen die dem Fachmann hinlänglich bekannten Vertreter dieser Substanzklasse in Frage, beispielsweise Alanin, β-Alanin, Arginin, Cystein, Cystin, Glycin, Histidin, Leucin, Isoleucin, Phenylalanin, Prolin etc..
Die Veresterung mit β-Alanin ist bevorzugt.

Gemäß einer Variante der Erfindung sind Verbindungen der allgemeinen Formel I bevorzugt,
worin
- R²: ein Wasserstoff- oder Halogenatom oder eine Hydroxygruppe
- R³: eine Gruppe R¹⁸-O-, R¹⁹SO₂-O- oder -O-C(O)R²², mit R¹⁸, R¹⁹ und R²² jeweils in der unter R² angegebenen Bedeutung;
- R⁶ und R⁷: je ein Wasserstoffatom;
- R^{6'}: ein Wasserstoffatom, ein Hydroxygruppe, eine Gruppe R²² in der unter R² angegebenen Bedeutung;
- R^{7'}: ein Wasserstoffatom, ein Halogenatom, eine Gruppe R¹⁸-O-, R¹⁹SO₂-O - oder -R²², mit R¹⁸, R¹⁹ und R²² jeweils in der unter R² angegebenen Bedeutung,
- R⁸: einen gerad- oder verzweigtkettigen, gegebenenfalls teilweise oder vollständig halogenierten Alkyl- oder Alkenylrest mit bis zu 5 Kohlenstoffatomen, einen Ethinyl- oder Prop-1-inylrest;
- R⁹: ein Wasserstoffatom oder zusammen mit R¹¹ eine zusätzliche Bindung;
- R¹¹: eine n-Pentyl- oder n-Hexylgruppe;
- R¹⁴, R¹⁵ und R¹⁶: jeweils ein Wasserstoffatom;
- R^{16'}: ein Wasserstoffatom, ein Halogenatom, eine Gruppe R¹⁸-O-, R¹⁹SO₂-O - oder -R²², mit R¹⁸, R¹⁹ und R²² jeweils in der unter R² angegebenen Bedeutung;
- R¹⁷ und R^{17'}: ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder -O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² angegebenen Bedeutung; sowie
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

Eine weitere bevorzugte Variante der vorliegenden Erfindung sieht die Verwendung solcher Verbindungen der allgemeinen Formel I' vor,
worin
- R²: ein Wasserstoff- oder Fluoratom oder eine Hydroxygruppe,
- R³: eine Gruppe R¹⁸-O-, R¹⁹SO₂-O- oder -O-C(O)R²², mit R¹⁸, R¹⁹ und R²² jeweils in der unter R² angegebenen Bedeutung;
- R⁶ und R⁷: jeweils ein Wasserstoffatom;
- R^{6'}: ein Wasserstoffatom oder eine Hydroxygruppe,
- R^{7'}: ein Wasserstoffatom, ein Fluor- oder Chloratom, eine Gruppe R¹⁸-O-, R¹⁹SO₂-O- oder -R²², mit R¹⁸, R¹⁹ und R²² jeweils in der unter R² angegebenen Bedeutung;
- R⁸: einen gerad- oder verzweigtkettigen, gegebenenfalls teilweise oder vollständig fluorierten Alkyl- oder Alkenylrest mit bis zu 5 Kohlenstoffatomen, einen Ethinyl- oder Prop-1-inylrest;
- R¹¹: eine n-Pentyl- oder n-Hexylgruppe;
- R¹⁴, R¹⁵ und R¹⁶: jeweils ein Wasserstoffatom;
- R^{16'}: ein Wasserstoffatom, ein Fluor- oder Chloratom oder eine Gruppe R¹⁸-O oder -R²², mit R¹⁸ und R²² jeweils in der unter R² angegebenen Bedeutung;
- R¹⁷ und R^{17'}: ein Wasserstoffatom und ein Halogenatom; ein Wasserstoffatom und eine Benzyloxygruppe; ein Wasserstoffatom und eine Gruppe R¹⁹SO₂-O-; eine Gruppe R¹⁸ und eine Gruppe -C(O)R²² oder -O-C(O)R²²; eine Gruppe R¹⁸-O- und eine Gruppe R¹⁸-; eine Gruppe R¹⁸-O- und eine Gruppe -O-C(O)R²², in allen vorstehenden Fällen mit R¹⁸, R¹⁹ und R²² jeweils in der unter R² angegebenen Bedeutung; oder
- R¹⁷ und R^{17'}: gemeinsam eine Gruppe =CR²³R²⁴, worin R²³ und R²⁴ unabhängig voneinander ein Wasserstoffatom und ein Halogenatom darstellen, oder gemeinsam ein Sauerstoffatom;
bedeuten.

Gemäß einer weiteren Variante betrifft die vorliegende Erfindung 8β-substituierte Estra-1,3,5(10)-trien-derivate der allgemeinen Formel I,
worin

Eine weitere Variante der Erfindung sind Estratrien-derivate der allgemeinen Formel I
worin
R¹⁷ und R^{17'} eine Gruppe R¹⁸-O- und eine Gruppe R¹⁸-; eine Gruppe R¹⁸- und eine Gruppe -O-C(O)R²², mit R¹⁸ und R²² jeweils in der unter R² angegebenen Bedeutung;
bedeuten.

Von diesen letztgenannten sind diejenigen bevorzugt
worin
R¹⁷ und R^{17'} eine Hydroxygruppe und ein Wasserstoffatom, eine C₁ - C₄-Alkyl- oder C₂ - C₄-Alkenylgruppe
ist
und insbesondere bevorzugt diejenigen
worin
R¹⁷ und R^{17'} eine Hydroxygruppe und ein Wasserstoffatom, eine Methyl-, Ethinyl- oder Prop-1-inylgruppe
ist.

Erfindungsgemäß bevorzugt sind die Verbindungen
8β-Methyl-11β-pentyl-1,3,5(10)-trien-3,17β-diol
8β-Ethyl-11β-pentyl-1,3,5(10)-trien-3,17β-diol
11β-Pentyl-8β-vinyl-estra-1,3,5(10)-trien-3,17β-diol
11β-Hexyl-8β-methyl-1,3,5(10)-trien-3,17β-diol
8β-Ethyl-11β-hexyl-1,3,5(10)-trien-3,17β-diol
11β-Hexyl-8β-vinyl-estra-1,3,5(10)-trien-3,17β-diol
8β-Methyl-11β-pentyl-1,3,5(10)-trien-3,17β-diol-3-sulfamat
8β-Ethyl-11β-pentyl-1,3,5(10)-trien-3,17β-diol-3-sulfamat
11β-Pentyl-8β-vinyl-estra-1,3,5(10)-trien-3,17β-diol-3-sulfamat
11β-Hexyl-8β-methyl-1,3,5(10)-trien-3,17β-diol-3-sulfamat
8β-Ethyl-11β-hexyl-1,3,5(10)-trien-3,17β-diol-3-sulfamat
11β-Hexyl-8β-vinyl-estra-1,3,5(10)-trien-3,17β-diol-3-sulfamat
8β-Methyl-11β-pentyl-1,3,5(10)-trien-3,17β-diol-3-acetat
8β-Ethyl-11β-pentyl-1,3,5(10)-trien-3,17β-diol-3-acetat
11β-Pentyl-8β-vinyl-estra-1,3,5(10)-trien-3,17β-diol-3-acetat
11β-Hexyl-8β-methyl-1,3,5(10)-trien-3,17β-diol-3-acetat
8β-Ethyl-11β-hexyl-1,3,5(10)-trien-3,17β-diol-3-acetat
11β-Hexyl-8β-vinyl-estra-1,3,5(10)-trien-3,17β-diol-3-acetat.

Die neuen Verbindungen sind zur Hemmung der Follikulogenese und der Ovulation, zur männlichen Kontrazeption und zur Behandlung von gutartigen und bösartigen proliferativen Erkrankungen des Ovars geeignet.
Anders als bei dem üblicherweise für die hormonelle Kontrazeption verwendeten Estrogen Ethinylestradiol oder auch bei den nach der WO 00/31112 für die Kontrazeption zu verwendenden Verbindungen können die erfindungsgemäßen Verbindungen der allgemeinen Formel I alleine, d. h. ohne die zusätzliche Gabe von Gestagenen zur Kontrazeption verwendet werden..

Die erfindungsgemäßen Ester der 8β-substituierten Estratriene können als Prodrugs Vorteile gegenüber den unveresterten Wirkstoffen hinsichtlich ihres Applikationsmodus, ihrer Wirkungsart, Wirkungsstärke und Wirkungsdauer aufweisen.

Pharmakokinetische und pharmakodynamische Vorteile weisen auch die erfindungsgemäßen Sulfamate der 8β-substituierten Estratriene auf. Diesbezügliche Effekte wurden bereits bei anderen Steroid-Sulfamaten beschrieben (J. Steroid Biochem. Molec. Biol, 55, 395 - 403 (1995); Exp. Opinion Invest. Drugs 7, 575 - 589 (1998)).

In der vorliegenden Patentanmeldung werden Steroide, denen das 8β-substituierte Estra-1,3,5(10)trien-Gerüst zugrunde liegt und die in 11-Stellung mit einer β-ständigen n-Pentyl- oder n-Hexylgruppe substituiert sind, zur Kontrazeption beschrieben, die in vitro Dissoziation hinsichtlich Bindung an Estrogenrezeptorpräparationen von Rattenprostata und Rattenuterus und die in vivo vorzugsweise eine Hemmung der Follikulogenese und der Ovulation aufweisen: über einen breiten Dosisbereich wirken diese Substanzen kontrazeptiv ohne andere Östrogen-sensitiven Organe wie z.B. den Uterus oder die Leber zu beeinflußen.

Darüberhinaus können diese Verbindungen zur männlichen Kontrazeption und zur Behandlung von gutartigen oder bösartigen proliferativen Erkrankungen des Ovars eingesetzt werden.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten zur Herstellung von Arzneimitteln, insbesondere für die nachstehenden Indikationen.

Die Verbindungen können, sowohl nach oraler als auch parenteraler Gabe, für die folgenden Indikationen eingesetzt werden.

Die im vorliegenden Patent beschriebenen neuartigen selektiven Estrogene können als Einzelkomponente in pharmazeutischen Zubereitungen oder in Kombination insbesondere mit GnRH-antagonisten, Progesteronrezeptor-antagonisten, Mesoprogestinen oder Gestagenen oder gewebeselektiver Gestagene (Wirkung über Typ A/B-Form) eingesetzt werden.

Die Substanzen und die sie enthaltenden Pharmaka sind besonders geeignet für die ovarielle Kontrazeption, für die Behandlung von gutartigen oder bösartigen proliferativen Erkrankungen des Ovars, wie z.B. Ovarialcarcinome, Granulosazelltumore.
Außerdem können die Verbindungen zur Behandlung männlicher Fertilitätsstörungen und prostatischer Erkrankungen Verwendung finden.

Die zu verabreichende Menge einer Verbindung der allgemeinen Formel I' schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 µg/kg - 100 mg/kg Körpergewicht, vorzugsweise 0,04 µg/kg - 1 mg/kg Körpergewicht, je Tag betragen.
Beim Menschen entspricht dies einer Dosis von 0,8 µg bis 8 g, vorzugsweise 3,2 µg bis 80 mg, täglich.

Eine Dosiseinheit enthält erfindungsgemäß 1,6 µg bis 2000 mg einer oder mehrerer Verbindungen der allgemeinen Formel I'.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.
Die Verbindungen lassen sich auch in Form einer Depotinjektion oder eines Implantatpräparats anwenden, die so formuliert sein können, daß eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B. Pessare, Spiralen, IUSs, Mirena®) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Erfindungsgemäß können die Verbindungen der allgemeinen Formel I auch mit Liposomen verkapselt werden.

### Methoden

### Estrogenrezeptorbindungsstudien

Die Bindungsaffinität der neuen selektiven Estrogene wurde in Kompetitionsexperimenten unter Verwendung von 3H-Estradiol als Ligand an Estrogenrezeptorpräparationen von Rattenprostata und Rattenuterus getestet. Die Präparation des Prostatacytosols und der Estrogenrezeptortest mit dem Prostatacytosol wurde, wie von Testas et al. (1981) beschrieben, durchgeführt (Testas J. et al., 1981, Endocrinology 109: 1287-1289).
Die Präparation von Rattenuteruscytosol, sowie der Rezeptortest mit dem ERhaltigen Cytosol wurden prinzipiell durchgeführt wie von Stack und Gorski, 1985, beschrieben (Stack, Gorski 1985, Endocrinology 117, 2024-2032) mit einigen Modifikationen wie bei Fuhrmann et al. (1995) beschrieben (Fuhrmann U. et al. 1995, Contraception 51: 45-52).

Die im vorliegenden Patent beschriebenen Substanzen weisen höhere Bindungsaffinität zu Estrogenrezeptor aus Rattenprostata als zu Estrogenrezeptor aus Rattenuterus auf. Dabei wird davon ausgegangen, daß ERβ gegenüber ERα in der Rattenprostata, in Rattenuterus ERα gegenüber ERβ überwiegt. Tabelle 1 zeigt, daß das Verhältnis der Bindung an Prostata- und Uterusrezeptor qualitativ mit dem Quotient der relativen Bindungsaffinität (RBA) an humanen ERβ und ERα von Ratte (nach Kuiper et al. (1996), Endocrinology 138: 863-870) übereinstimmt (Tabelle 1).

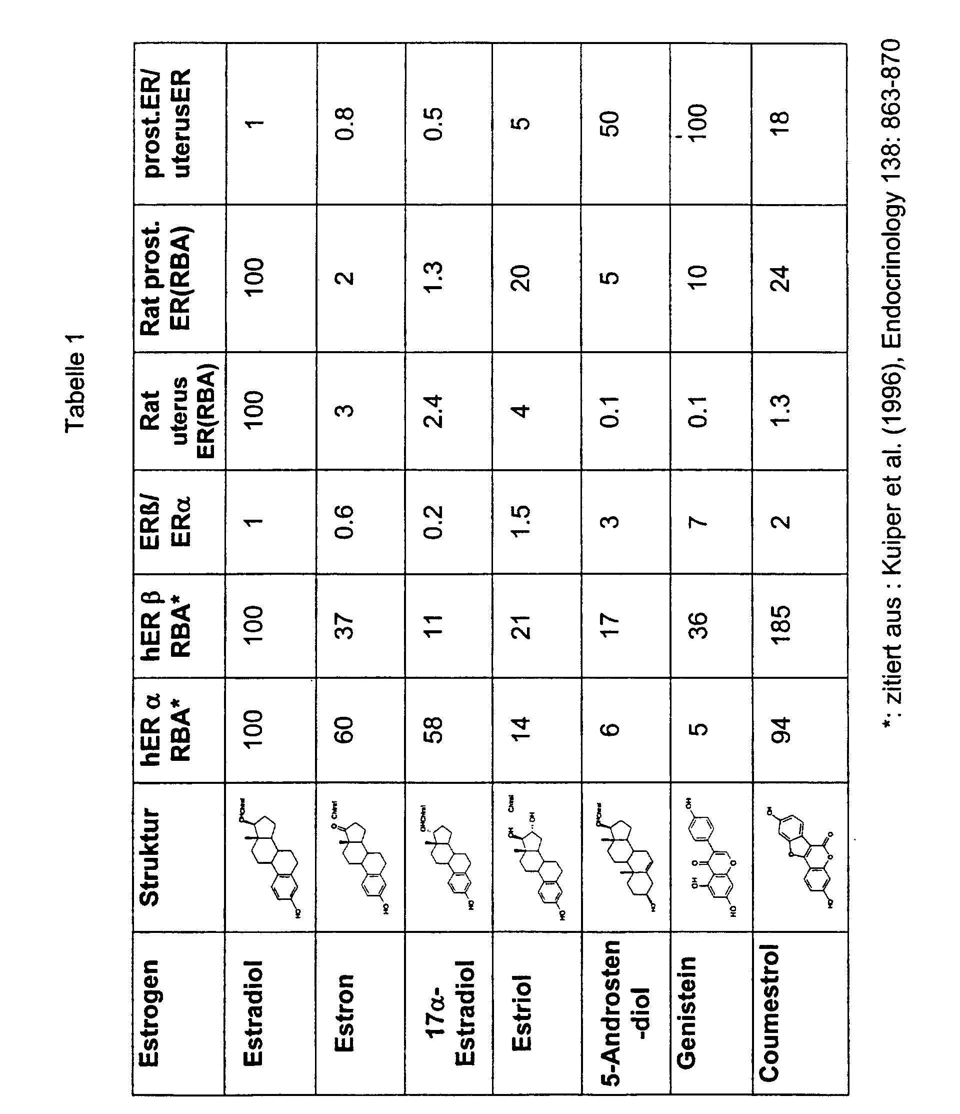

### Untersuchungsbeispiele zur kontrazeptiven Wirkung

### (a) Untersuchung der frühen Follikulogenese:

Immature weibliche Ratten werden hypophysektomiert. Dieser Tag wird als Tag 0 definiert. Von Tag 1 - Tag 4 erfolgt Behandlung, subcutan oder/und oral, mit der Wirksubstanz in Kombination mit 17β-Östradiol. Autopsie der Tiere erfolgt am Tag 5. Das Ovar wird entnommen und makroskopisch, z.B. Organgewichte, und mikroskopisch, z.B. histologische Beurteilung der Follikel, sog. Follikelstaging, analysiert.

### (b) Untersuchung der späten Follikulogenese /Ovulation

Immature weibliche Ratten werden hypophysektomiert. Dieser Tag wird als Tag 0 definiert. Von Tag 1 - Tag 4 erfolgt Behandlung, subcutan oder/und oral, mit der Wirksubstanz in Kombination mit 17β-Östradiol. Am Tag 5 erfolgt eine subkutane Injektion mit PMSG (pregnant mare serum gonadotropin). Am Tag 7 wird hCG intraperitoneal zur Auslösung der Ovulation appliziert. Am Tag 8 wird das Ovar entnommen und makroskopisch (z.B. Ovargewichte) und/oder mikroskopisch (z.B. histologische Beurteilung der Follikel, sogenanntes Follikelstaging) analysiert. Die Tuben werden gespült und auf die Anwesenheit von Eizellen untersucht.

### (c) Untersuchung der Ovulation

Immature weibliche Ratten werden im Alter von 23 Tagen subkutan mit PMSG (pregnant mare serum gonadotropin) behandelt (Tag 1). Am selben Tag, sowie 24 und 48 Stunden später erhalten die Tiere die Wirksubstanz subkutan oder oral appliziert. 54 Stunden nach der PMSG Injektion erhalten die Tiere zur Auslösung der Ovulation eine intraperitoneale Injektion von hCG. Autopsie erfolgt 16 Stunden nach der hCG-Gabe. Die Tuben werden gespült und auf die Anwesenheit von Eizellen hin untersucht.

Eine andere Möglichkeit, die dissoziierte Estrogenwirkung der erfindungsgemäßen Substanzen in vivo nachzuweisen, besteht darin, nach Einmalapplikation der Substanzen bei Ratten Effekte auf die Expression von 5HT2a-Rezeptor- und Serotonintransporter-Protein- und mRNA-Level in ERβ-reichen Gehirnarealen zuvermessen. Vergleichend zum Effekt auf Serotoninrezeptor- und Transporterexpression wird der Effekt auf die LH-Sekretion gemessen. Substanzen mit höherer Bindung an den Rattenprosta- verglichen mit dem Rattenuterusestrogenrezeptor sind potenter hinsichtlich Erhöhung der Expression von Serotoninrezeptor- und transporter, im Vergleich zu ihrem positiven Effekt auf die LH-Ausschüttung. Die Dichte von Serotoninrezeptor und -Transporter wird an Gehirnschnitten mittels radioaktiver Liganden, die entsprechende mRNA mittels in situ Hybridisierung bestimmt. Die Methode ist in der Literatur beschrieben: G. Fink & B.E.H. Sumner 1996 Nature 383:306; B. E.H. Sumner et al. 1999 Molecular Brain Research, in press.

### Herstellung der erfindungsgemäßen Verbindungen

Die erfindungsgemäßen Verbindungen der allgemeinen Formel I werden wie in den Beispielen beschrieben hergestellt. Durch analoge Vorgehensweise unter Verwendung homologer Reagenzien zu den in den Beispielen beschriebenen Reagenzien lassen sich weitere Verbindungen der allgemeinen Formel I erhalten.

Veretherung und/oder Veresterung freier Hydroxygruppen erfolgt nach dem Fachmann gängigen Methoden.

Die erfindungsgemäßen Verbindungen können an den Kohlenstoffatomen 6, 7, 15, 16 und 17 als α,β-Stereoisomere vorliegen. Bei der Herstellung der Verbindungen gemäß den beschriebenen Verfahren fallen die Verbindungen meist als Gemische der entsprechenden α,β-Isomeren an. Die Gemische lassen sich beispielsweise durch chromatographische Verfahren trennen.

Gemäß der allgemeinen Formel I mögliche Substituenten können bereits in der endgültigen Form oder in Form eines Vorläufers schon im Ausgangsprodukt, einem bereits dem gewünschten Endprodukt entsprechend substituierten Estron, vorhanden sein.

So ist die Einführung eines Substituenten bzw. reaktiven Vorläufers am Kohlenstoffatom 7 durch nukleophile Addition des Substituenten bzw. Vorläufers an ein 6-Vinylsulfon möglich (DE 42 18 743 A1). Hierbei werden in unterschiedlichen Anteilen, abhängig von den Reaktionspartnern und den gewählten Reaktionsbedingungen, 7α- und 7β-substituierte Verbindungen erhalten, die sich beispielsweise durch chromatographische Verfahren trennen lassen.

17-Substituenten werden, ebenfalls nach bekannten Verfahren, durch nukleophile Addition des gewünschten Substituenten oder eines reaktiven Vorläufers davon, eingeführt und gegebebenenfalls weiter aufgebaut.

Die erfindungsgemäßen 8β-substituierten Estratrien-Carbonsäureester werden in Analogie zu ebenfalls bekannten Verfahren aus den entsprechenden Hydroxysteroiden hergestellt (siehe z.B. Pharmazeutische Wirkstoffe, Synthesen, Patente, Anwendungen; A. Kleemann, J. Engel', Georg Thieme Verlag Stuttgart 1978. Arzneimittel, Fortschritte 1972 bis 1985; A. Kleemann, E. Lindner, J. Engel (Hrsg.), VCH 1987, S. 773-814).

Die erfindungsgemäßen Estratrien-Sulfamate sind in an sich bekannter Weise aus den entsprechenden Hydroxy-Steroiden durch Veresterung mit Sulfamoylchloriden in Gegenwart einer Base zugänglich (Z. Chem. **15**, 270-272 (1975); Steroids **61**, 710 - 717 (1996)).
Nachfolgende Acylierung der Sulfamidgruppe führt zu den erfindungsgemäßen (N-Acyl)sulfamaten, für die bereits im Falle der Abwesenheit eines 8-Substituenten pharmakokinetische Vorteile nachgewiesen wurden (vgl. DE 195 40 233 A1).

Die regioselektive Veresterung von polyhydroxylierten Steroiden mit N-substituierten und N-unsubstituierten Sulfamoylchloriden erfolgt nach partiellem Schutz derjenigen Hydroxylgruppen, die unverestert bleiben sollen. Als Schutzgruppen mit hierfür geeigneter selektiver Reaktivität haben sich Silylether erwiesen, da diese unter den Bedingungen der Sulfamatbildung stabil sind und die Sulfamatgruppe intakt bleibt, wenn die Silylether zur Regenerierung der restlichen im Molekül noch enthaltenen Hydroxylgruppe(n) wieder abgespalten werden (Steroids **61**, 710 - 717 (1996)).
Die Herstellung der erfindungsgemäßen Sulfamate mit einer oder mehreren zusätzlichen Hydroxylgruppen im Molekül ist auch dadurch möglich, daß man von geeigneten Hydroxy-Steroidketonen ausgeht. Zunächst werden, je nach Zielstellung, eine oder mehrere vorhandene Hydroxylgruppen einer Sulfamoylierung unterworfen. Dann können die Sulfamatgrupen gegebenenfalls mit einem gewünschten Acylchlorid in Gegenwart einer Base in die betreffenden /N-Acyl)sulfamate überführt werden. Die nunmehr vorliegenden Oxosulfamate oder Oxo-(N-acyl)sulfamate werden durch Reduktion in die entsprechenden Hydroxysulfamate bzw. Hydroxy-(N-acyl)sulfamate umgewandelt (Steroids **61**, 710 - 717 (1996)). Als geeignete Reduktionsmittel kommen Natriumborhydrid und der Boran-Dimethylsulfid-Komplex in Frage.

Funktionalisierungen am Kohlenstoffatom 2 sind beispielsweise durch elektrophile Substitution nach vorheriger Deprotonierung der Position 2 des entsprechenden 3-(2-Tetrahydropyranyl)- oder 3-Methylethers mit einer Lithium-Base (z. B. Methyllithium, Butyllithium) möglich. So kann zum Beispiel ein Fluoratom durch Umsetzung des C-H-aktivierten Substrats mit einem Flourierungsreagenz wie N-Fluormethansulfonimid (WO 94/24098) eingeführt werden.

Die Einführung variabler Substituenten in die Ringe B, C und D des Estratriengerüstes kann prinzipiell nach der dem Fachmann bekannten chemischen Lehre erfolgen, mit der die entsprechenden, in 8-Stellung nicht substituierten Estratrienderivate hergestellt werden (siehe unter anderem: Steroide, L.F. Fieser, M. Fieser, Verlag Chemie, Weinheim / Bergstr., 1961; Organic Reactions in Steroid Chemistry, J. Fried, J.A. Edwards, Van Nostrand Reinhold Company, New York, Cincinnati, Toronto, London, Melbourne, 1972; Medicinal Chemistry of Steroids, F.J. Zeelen, Elsevier, Amsterdam, Oxford, New York, Tokyo, 1990). Das betrifft beispielsweise die Einführung von Substituenten, wie Hydroxyl- oder Alkyloxygruppen, Alkyl, Alkenyl- oder Alkinylgruppen oder Halogen, insbesondere Fluor.

Substituenten gemäß der allgemeinen Formel I können aber auch auf der Stufe der bereits in 8-Stellung substituierten Estratriene eingeführt werden. Dies kann insbesondere bei Mehrfachsubstitution der gewünschten Endverbindung sinnvoll bzw. erforderlich sein.

Die nachfolgenden Beispiele dienen der näheren Erläuterung der Erfindung.

Als Ausgangsmaterial für derartige Synthesen dienen 11-Keto-estratetraenderivate (US 3491089, Tetrahedron Letters, 1967, 37, 3603.), welche bei der Umsetzung mit Diethylaluminiumcyanid stereoselektiv in Position 8β substitiuiert werden. Durch anschließende Reduktion der Carbonylfunktion an C(11) und Eliminieren der entstandenen Hydroxylgruppe gelangt man zu 8β-substituierten Estra-1,3,5(10),9(11)-tetraenen, die wiederum in 8β-Aldehyde überführbar sind. Eine Funktionalisierung, z.B. durch Wittig-Reaktionen mit nachfolgendem Entfernen der Schutzgruppen, führt zu den erfindungsgemäßen 8β-Steroiden.

Die bei dieser Sequenz zunächst erhaltenen 11-oxygenierten Estradiolderivate lassen sich, wie auch die Doppelbindung C(9)-C(11), nach dem Fachmann bekannten Methoden weiter zu vielfältigen Substitutionsmustern am Steroid umsetzen. Beispielsweise kann eine 11α-Hydroxygruppe nach dem von Vorbrüggen et al. beschriebenen Verfahren in ein 11β-Fluoratom überführt werden.

Für die Herstellung der erfindungsgemäßen Derivate der 8β-substituierten Estra-1,3,5(10)-trien-3,16ξ-diole ohne 17-Substituenten findet vor allem die folgende Synthesestrategie Verwendung. Hierbei wird die 8β-Carbonylfunktion als Acetal geschützt. Nach anschließender Oxidation, kann das 17-Ketosteroid in ein Sulfonylhydrazon überführt werden, im einfachsten Falle durch Umsetzung mit Phenylsulfonylhydrazid. Durch eine Abbaureaktion erfolgt die Bildung des C(16)-C(17) Olefins (Z. Chem. 1970, 10, 221-2; Liebigs Ann. Chem. 1981, 1973-81), an das in regio/stereokontrollierter Weise Hypobromid angelagert wird. Reduktive Dehalogenierung und Entfernung der Acetalschutzgruppe an 8β geben den Weg für Transformationen zu den erfindungsgemäßen Verbindungen frei. Die nach dieser Art erhältlichen 16β-Alkohole können durch bekannte Methoden in das 16α-Epimer überführt werden (Synthesis 1980, 1)

Eine weitere Variante für die Einführung der Hydroxylgruppe an C-Atom 16 besteht in der Hydroborierung der 16(17)-Doppelbindung mit sterisch anspruchsvollen Boranen. Von dieser Reaktion ist bekannt, daß sie zu 16-oxygenierten Produkten führt (Indian J. Chem. 1971, 9, 287-8). Dementsprechend ergibt die Umsetzung der Estra-1,3,5(10),16-tetraene mit 9-Borabicyclo[3.3.1]nonan nach der Oxidation mit alkalischem Wasserstoffperoxid 16α-Hydroxyestratriene. In untergeordnetem Maße werden bei dieser Reaktion die epimeren 16β-Hydroxysteroide gebildet. Weitere Transformationen am 8β -Substituenten führen dann zu den erfindungsgemäßen Verbindungen der allgemeinen Formel I.

Charakteristische, aber nicht einschränkende Syntheseverfahren, die zur Schaffung repräsentativer Substitutionsmuster am Estrongerüst, auch in Kombination zu mehreren Substituenten, nützlich sind, finden sich etwa in: C(1) J. Chem. Soc. (C)1968, 2915; C(7) Steroids 54, 1989, 71; C(8α) Tetrahedron Letters 1991, 743; C(8β) Tetrahedron Letters 1964, 1763; J. Org. Chem. 1970, 35, 468; C(11) J. Steroid Biochem. 31, 1988, 549; Tetrahedron 33, 1977, 609 und J. Org. Chem. 60, 1995, 5316; C(9) DE-OS 2035879; J. Chem. Soc. Perk. 1 1973, 2095; C(15) J. Chem. Soc. Perk.1 1996, 1269.); C(13α) Mendeleev Commun. 1994, 187; C(14β) Z. Chem. 23, 1983,410.

In den Beispielen und den Schemata gelten die folgenden Abkürzungen:
THF = Tetrahydrofuran; THP = Tetrahydropyran-2-yl; DHP = Dihydropyran;
DMSO = Dimethylsulfoxid; MTBE = Methyl-tert.-butylether; DIBAH = Diisobutylaluminiumhydrid; LTBAH = Lithium-tri-tert.-butoxyaluminiumhydrid;

### Beispiel 1

### 8β-Formyl-3-methoxy-17β-(tetrahydropyran-2-yloxy)-9β-estra-1,3,5(10)-trien-11-ol (2)

Zu 4.36 g des 8β-Cyanosteroids **1** in 105 ml abs. Toluol wurden bei 0°C 9.2 ml DIBAH in 32 ml abs. Toluol getropft und 2 h bei dieser Temperatur gerührt. Die Reaktionslösung wurde nacheinander mit 215 ml Toluol, 32 ml ges. Natriumhydrogencarbonatlösung und 4 ml *iso*-Propanol versetzt und über Nacht nachgerührt. Anschließend wurde der ausgefallene Niederschlag abgesaugt, das Filtrat mit Wasser und ges. Natriumchloridlösung gewaschen, mit Magnesiumsulfat getrocknet und im Vakuum eingeengt. Der ölige Rückstand wurde an Kieselgel (Cyclohexan/Essigester 2:1) gereinigt und 3.76 g **2** wurden als farbloser Schaum erhalten.

### 3-Methoxy-8β-methyl-17β-(tetrahydropyran-2-yloxy)-9β-estra-1,3,5(10)-trien-11-ol (3)

Zu einer Lösung von 3.44 g Kaliumhydroxid in 60 ml Triethylenglykol wurden bei Raumtemperatur 2.45 ml Hydraziniumhydroxid (80 %ig, mit Wasser) und 1.02 g 8β-Formylsteroid **2** in 100 ml Triethylenglykol gegeben und 3 h auf 200° C erwärmt. Nach dem Abkühlen wurde die Reaktionslösung mit 160 ml Wasser versetzt und mit 10 %iger Schwefelsäure neutralisiert. Das Gemisch wurde mehrmals mit Diethylether extrahiert, die organischen Phasen mit Wasser und ges. Natriumchloridlösung gewaschen, mit Magnesiumsulfat getrocknet und am Rotationsverdampfer zur Trockene eingeengt. Die so erhaltenen 965 mg schaumförmigen 8β-Methylsteroids **3** wurden ohne weitere Reinigung in der nächsten Stufe eingesetzt.

### 3-Methoxy-8β-methyl-17β-(tetrahydropyran-2-yloxy)-9β-estra-1,3,5(10)-trien-11-on (4)

Zu einer Lösung von 965 mg Alkohol **3** in 24 ml Dichlormethan wurden 1.06 g PCC gegeben und für 2 h bei Raumtemperatur nachgerührt. Die Reaktionslösung wurde mittels einer kurzen Fritte über Kieselgel vom Niederschlag abfiltriert und im Vakuum eingeengt. Der Rückstand wurde an Kieselgel (Cyclohexan/Essigester 4:1) gereinigt und 671 mg **4** als farbloser Schaum erhalten.

### Allgemeine Arbeitsvorschrift zur Einführung der 11-Alkylkette in 3-Methoxy-17β-(tetrahydropyran-2-yloxy)-estra-1,3,5(10)-trien-11-onen

Zu einer Suspension von 15 eq. wasserfreiem Cer(III)-chlorid in 5 ml/mmol abs. THF wurden unter Argon bei -78°C 10 eq. der entsprechenden Alkyllithiumverbindung getropft. Nach 30 min bei -78°C wurde zum frisch hergestellten Cer-Reagens eine Lösung von 1 mmol 11-Ketosteroid in 5 ml abs. THF dazugetropft. Die Reaktion wurde bis zum vollständigen Umsatz bei -70- -40°C gerührt. Anschließend wird die Reaktionslösung mit ges. Ammoniumchloridlösung/Wasser/Diethylether (1:1:1) versetzt und die Phasen getrennt. Die wässrige Phase wurde mehrmals mit Ether extrahiert, die vereinigten organischen Phasen mit Wasser und ges. Natriumchloridlösung gewaschen, mit Magnesiumsulfat getrocknet und im Vakuum eingeengt. Der ölige Rückstand wurde an Kieselgel säulenchromatographisch getrennt.

### 3-Methoxy-8β-methyl-11β-pentyl-17β-(tetrahydropyran-2-yloxy)-9β-estra-1,3,5(10)-trien-11α-ol (5)

200 mg 11-Ketosteroid **4** wurde mit CeCl₃/ nPentLi (1 M in Diethylether) nach der allgemeinen Arbeitsvorschrift 1.4 umgesetzt. Säulenchromatographie (Cyclohexan/Essigester 5:1) ergab 224 mg farblosen Schaum **5.**

### 3-Methoxy-8β-methyl-11-pentyl-1,3,5(10),9(11)-tetraen-17β-ol (7)

Zu einer Lösung von 120 mg **5** in 2.5 ml Toluol wurden bei Raumtemperatur 132 mg *p*-Toluolsulfonsäure gegeben. Die Reaktionslösung wurde für 90 min auf 80°C erwärmt und nach Abkühlung auf Raumtemperatur mit Wasser versetzt. Die wässrige Phase wurde mehrmals mit Toluol extrahiert, die vereinigten organischen Phasen mit Wasser, ges. Natriumhydrogencarbonat- und ges. Natriumchlorid-Lösung gewaschen, über Magnesiumsulfat getrocknet und im Vakuum eingeengt. Chromatographische Reinigung an Kieselgel (Cyclohexan/Essigester 5:1) ergaben 64 mg eines Gemisches (Verhältnis 1.6:1) an Δ9,11-Steroid **7** und dem entsprechenden Steroid mit *exo*-cyclischer Doppelbindung. Auftrennung dieses Gemisches erfolgte mittels präparativer HPLC (Acetonitril/Wasser 9:1) und ergab das Δ9,11-Steroid **7** als farblosen Feststoff (Fp: 148-149°C).

### 3-Methoxy-8β-methyl-11β-pentyl-1,3,5(10)-trien-17β-ol (9)

Eine Lösung von 35 mg 7 in 1.9 ml Tetrahydrofuran/Methanol (1:1) wurde mit 47 mg Palladium (10%ig, auf Kohle) versetzt und unter Wasserstoffatmosphäre für ca. 2 Wochen bei Raumtemperatur gerührt. Die Raktionslösung wurde über Celite vom Katalysator abfiltriert und im Vakuum eingeengt. Der erhaltene farblose Schaum **9** (ca. 25 mg) wurde ohne weitere Reinigung in der nächsten Stufe verwendet.

### 8β-Methyl-11β-pentyl-1,3,5(10)-trien-3,17β-diol (11)

Zu einer Lösung von 25 mg 3-Methylether **9** in 1.35 ml abs. Toluol wurden bei 0°C 0.15 ml DIBAH zugetropft. Anschließend wurde die Reaktionslösung für 4 Stunden unter Rückfluss erhitzt. Nach erneuter Abkühlung auf 0°C wurden nacheinander je 0.68 ml Ethanol, Ethanol/Wasser (1:1) und halbkonz. Salzsäure dazugegeben. Nach erfolgter Phasentrennung wurde die wässrige Phase mehrmals mit Toluol extrahiert, die vereinigten organischen Phasen mit Wasser, ges. Natriumhydrogencarbonat- und ges. Natriumchlorid-Lösung gewaschen, über Magnesiumsulfat getrocknet und im Vakuum eingeengt. Die säulenchromatographische Reinigung erfolgte an Kieselgel (Cyclohexan/Essigester 3:1) und ergab 15 mg farblosen Feststoff **11** (Fp: 150-152°C).

### Beispiel 2

Die Synthese von Substanz **4** wurde unter Beispiel 1, 1.1-1.3 beschrieben.

### 11β-Hexyl-3-methoxy-8β-methyl-17β-(tetrahydropyran-2-yloxy)-9β-estra-1,3,5(10)-trien-11α-ol (6)

166 mg 11-Ketosteroid **4** wurde mit CeCl₃/ nHexLi (2.5 M in Hexan) nach der allgemeinen Arbeitsvorschrift 1.4 umgesetzt. Säulenchromatographie (Cyclohexan/Essigester 5:1) ergab 199 mg farblosen Schaum **6.**

### 11-Hexyl-3-methoxy-8β-methyl-1,3,5(10),9(11)-tetraen-17β-ol (8)

Zu einer Lösung von 76 mg **6** in 1.6 ml Toluol wurden bei Raumtemperatur 81 mg *p*-Toluolsulfonsäure gegeben. Die Reaktionslösung wurde für 90 min auf 80°C erwärmt und nach Abkühlung auf Raumtemperatur mit Wasser versetzt. Die wässrige Phase wurde mehrmals mit Toluol extrahiert, die vereinigten organischen Phasen mit Wasser, ges. Natriumhydrogencarbonat- und ges. Natriumchlorid-Lösung gewaschen, über Magnesiumsulfat getrocknet und im Vakuum eingeengt. Chromatographische Reinigung an Kieselgel (Cyclohexan/Essigester 5:1) ergaben 40 mg eines Gemisches (Verhältnis 1.3:1) an Δ9,11-Steroid **8** und dem entsprechenden Steroid mit exo-cyclischer Doppelbindung. Auftrennung dieses Gemisches erfolgte mittels präparativer HPLC (Acetonitril/Wasser 9:1) und ergab das Δ9,11-Steroid **8** als farblosen Feststoff (Fp: 147-149°C).

### 11β-Hexyl-3-methoxy-8β-methyl-1,3,5(10)-trien-17β-ol (10)

Eine Lösung von 15 mg **8** in 0.78 ml Tetrahydrofuran/Methanol (1:1) wurde mit 20 mg Palladium (10%ig, auf Kohle) versetzt und unter Wasserstoffatmosphäre für ca. 2 Wochen bei Raumtemperatur gerührt. Die Raktionslösung wurde über Celite vom Katalysator abfiltriert und im Vakuum eingeengt. Der erhaltene farblose Schaum **10** (ca. 10 mg) wurde ohne weitere Reinigung in der nächsten Stufe verwendet.

### 11β-Hexyl-8β-methyl-1,3,5(10)-trien-3,17β-diol (12)

Zu einer Lösung von 10 mg 3-Methylether **10** in 0.52 ml abs. Toluol wurden bei 0°C 0.06 ml DIBAH zugetropft. Anschließend wurde die Reaktionslösung für 4 Stunden unter Rückfluss erhitzt. Nach erneuter Abkühlung auf 0°C wurden nacheinander je 0.26 ml Ethanol, Ethanol/Wasser (1:1) und halbkonz. Salzsäure dazugegeben. Nach erfolgter Phasentrennung wurde die wässrige Phase mehrmals mit Toluol extrahiert, die vereinigten organischen Phasen mit Wasser, ges. Natriumhydrogencarbonat- und ges. Natriumchlorid-Lösung gewaschen, über Magnesiumsulfat getrocknet und im Vakuum eingeengt. Die säulenchromatographische Reinigung erfolgte an Kieselgel (Cyclohexan/Essigester 3:1) und ergab 5 mg farblosen Feststoff **12** (Fp: 152-154°C).

### Beispiel 3

### 8β-Cyano-3-methoxy-11β-pentyl-17β-(tetrahydropyran-2-yloxy)-estra-1,3,5(10)-trien-11α-ol (14)

300 mg 11-Ketosteroid **13** wurde mit CeCl₃/ nPentLi (1 M in Diethylether) nach der allgemeinen Arbeitsvorschrift 1.4 umgesetzt. Säulenchromatographie (Cyclohexan/Essigester 5:1) ergab 317 mg farblosen Schaum **14.**

### 8β-Cyano-3-methoxy-11-pentyl-estra-1,3,5(10),9(11)-tetraen-17β-ol (16)

Zu 270 mg 11-Hydroxysteroid **14** in 5.4 ml abs. Pyridin wurden bei 0°C 0.54 ml POCl₃ zugetropft. Das Kältebad wurde entfernt und die Reaktion für 12 Stunden auf 60°C erwärmt. Anschließend wurde die Reaktionslösung in eine eisgekühlte ges. Natriumhydrogencarbonatlösung getropft. Es wurde mit Wasser verdünnt und mehrmals mit Dichlormethan extrahiert. Die vereinigten organischen Phasen wurden mit ges. Natriumchloridlösung gewaschen, über Magnesiumsulfat getrocknet und im Vakuum eingeengt. Die Reinigung erfolgte säulenchromatographisch (Cyclohexan/Essigester 3:1). Man erhält 160 mg Tetraenol **16** als farblosen Schaum.

### 8β-Formyl-3-methoxy-11-pentyl-estra-1,3,5(10),9(11)-tetraen-17β-ol (18)

150 mg Nitril **16** wurden in 7.9 ml abs. Toluol gelöst und auf -10°C gekühlt. Anschließend wurden 1.19 ml DIBAH (1M in Toluol) zugetropft und die Reaktionslösung bis zum vollständigen Umsatz bei 0°C gerührt. Zur Aufarbeitung wurde die Reaktionslösung mit 8 ml Toluol verdünnt und bei 0°C 1.2 ml ges.

Natriumhydrogencarbonatlösung sowie 0.15 ml *iso*-Propanol zugetropft. Der kristalline Niederschlag wurde über Celite abfiltriert und die Lösung eingeengt.
Das so erhaltene rohe Imin wurde in 4 ml Ethanol/Wasser (5:1) gelöst und mit 376 mg *p*Toluolsulfonsäure versetzt. Die Reaktionslösung wurde bis zum vollständigen Umsatz auf 60°C erwärmt. Anschließend wurde die Reaktionslösung eingeengt, der Rückstand in Essigester aufgenommen und mehrmals mit Wasser, ges. Natriumhydrogencarbonatlösung und ges. Natriumchloridlösung gewaschen, über Magnesiumsulfat getrocknet und eingeengt. Nach säulenchromatographischer Reinigung (Cyclohexan/Essigester 3:1) wurden 83 mg Aldehyd **18** als farbloser Schaum erhalten.

### 8β-Formyl-3-methoxy-11β-pentyl-estra-1,3,5(10)-trien-17β-ol (20)

80 mg Tetraenol **18** in 4.2 ml Tetrahydrofuran/Methanol (1:1) wurden mit 105 mg Palladium (10%ig, auf Kohle) versetzt und für 2 Wochen bei Raumtemperatur gerührt. Anschließend wurde über Celite filtriert und die so erhaltenen 80 mg Trienol **20** ohne weitere Reinigung in der nächsten Stufe eingesetzt.

### 3-Methoxy-11β-pentyl-8β-vinyl-estra-1,3,5(10)-trien-17β-ol (22)

89 mg Natriumhydrid (80%) in 1.5 ml abs. Dimethylsulfoxid wurden für 1 Stunde auf 70°C erwärmt. Die erhaltene grauschwarze Lösung wurde bei RT zu einer Lösung von 1.12 g Methyltriphenylphosphoniumbromid in 6.2 ml abs. Dimethylsulfoxid getropft. Die Lösung verfärbte sich gelbgrün und wurde für eine weitere Stunde bei RT gerührt.
Eine Lösung von 80 mg des Aldehyds **20** in 1 ml abs. Dimethylsulfoxid wurde bei RT zur Lösung des Ylids zugetropft. Die Reaktionslösung wurde für 2 Stunden bei 40°C gerührt, mit Wasser bei 0°C versetzt und mehrmals mit Diethylether extrahiert. Die vereinigten organischen Phasen wurden mit Wasser und ges. Natriumchloridlösung gewaschen, über Magnesiumsulfat getrocknet und eingeengt. Säulenchromatographische Reinigung (Cyclohexan/Essigester 3:1) ergab 64 mg **22** als farbloser Feststoff (Fp:139-141 °C ).

### 11β-Pentyl-8β-vinyl-estra-1,3,5(10)-trien-3,17β-diol (24)

Zu einer Lösung von 60 mg 3-Methylether **22** in 3.1 ml abs. Toluol wurden bei 0°C 0.36 ml DIBAH getropft. Anschließend wurde für 4 Stunden unter Rückfluss erhitzt. Nach Abkühlen der Reaktionslösung auf 0°C wurde diese nacheinander mit 1.6 ml Ethanol, 1.6 ml Ethanol/Wasser (1:1) und 1.6 ml halbkonz. Salzsäure versetzt, anschließend mehrmals mit Essigester extrahiert, die vereinigten organischen Phasen mit Wasser und ges. Natriumchloridlösung gewaschen, über Magnesiumsulfat getrocknet und eingeengt. Säulenchromatographie (Cyclohexan/Essigester 2:1) des Rückstandes ergab 46 mg **24** als farblosen Feststoff (Fp: 144-146°C).

### Beispiel 4

### 8β-Cyano-11β-hexyl-3-methoxy-17β-(tetrahydropyran-2-yloxy)-estra-1,3,5(10)-trien-11α-ol (15)

300 mg 11-Ketosteroid **13** wurde mit CeCl₃/ nHexLi (2.5 M in Hexan) nach der allgemeinen Arbeitsvorschrift 1.4 umgesetzt. Säulenchromatographie (Cyclohexanl Essigester 5:1) ergab 352 mg farblosen Schaum **15.**

### 8β-Cyano-11-hexyl-3-methoxy-estra-1,3,5(10),9(11)-tetraen-17β-ol (17)

Zu 300 mg 11-Hydroxysteroid **15** in 6 ml abs. Pyridin wurden bei 0°C 0.6 ml POCl₃ zugetropft. Das Kältebad wurde entfernt und die Reaktion für 12 Stunden auf 60°C erwärmt. Anschließend wurde die Reaktionslösung in eine eisgekühlte ges. Natriumchloridlösung getropft. Es wurde mit Wasser verdünnt und mehrmals mit Dichlormethan extrahiert. Die vereinigten organischen Phasen wurden mit ges. Natriumchloridlösung gewaschen, über Magnesiumsulfat getrocknet und im Vakuum eingeengt. Die Reinigung erfolgte säulenchromatographisch (Cyclohexan/Essigester 3:1). Man erhält 190 mg Tetraenol **17** als farblosen Schaum.

### 8β-Formyl-11-hexyl-3-methoxy-estra-1,3,5(10),9(11)-tetraen-17β-ol (19)

170 mg Nitril **17** wurden in 8.6 ml abs. Toluol gelöst und auf -10°C gekühlt. Anschließend wurden 1.29 ml DIBAH (1M in Toluol) zugetropft und die Reaktionslösung bis zum vollständigen Umsatz bei 0°C gerührt. Zur Aufarbeitung wurde die Reaktionslösung mit 8.6 ml Toluol verdünnt und bei 0°C 1.3 ml ges. Natriumhydrogencarbonatlösung sowie 0.16 ml *iso*-Propanol zugetropft. Der kristalline Niederschlag wurde über Celite abfiltriert und die Lösung eingeengt.
Das so erhaltene rohe Imin wurde in 4.3 ml Ethanol/Wasser (5:1) gelöst und mit 411 mg *p*Toluolsulfonsäure versetzt. Die Reaktionslösung wurde bis zum vollständigen Umsatz auf 60°C erwärmt. Anschließend wurde die Reaktionslösung eingeengt, der Rückstand in Essigester aufgenommen und mehrmals mit Wasser, ges. Natriumhydrogencarbonatlösung und ges. Natriumchloridlösung gewaschen, über Magnesiumsulfat getrocknet und eingeengt. Nach säulenchromatographischer Reinigung (Cyclohexan/Essigester 3:1) wurden 86 mg Aldehyd **19** als farbloser Schaum erhalten.

### 8β-Formyl-11β-hexyl-3-methoxy-estra-1,3,5(10)-trien-17β-ol (21)

85 mg Tetraenol **19** in 4.2 ml Tetrahydrofuran/Methanol (1:1) wurden mit 105 mg Palladium (10%ig, auf Kohle) versetzt und für 2 Wochen bei Raumtemperatur gerührt. Anschließend wurde über Celite filtriert und die so erhaltenen 85 mg Trienol **21** ohne weitere Reinigung in der nächsten Stufe eingesetzt.

### 11β-Hexyl-3-methoxy-8β-vinyl-estra-1,3,5(10)-trien-17β-ol (23)

88 mg Natriumhydrid (80%) in 1.5 ml abs. Dimethylsulfoxid wurden für 1 Stunde auf 70°C erwärmt. Die erhaltene grauschwarze Lösung wurde bei RT zu einer Lösung von 1.10 g Methyltriphenylphosphoniumbromids in 6.2 ml abs. Dimethylsulfoxid getropft. Die Lösung verfärbte sich gelbgrün und wurde für eine weitere Stunde bei RT gerührt.
Eine Lösung von 82 mg des Aldehyds **21** in 1 ml abs. Dimethylsulfoxid wurde bei RT zur Lösung des Ylids zugetropft. Die Reaktionslösung wurde für 2 Stunden bei 40°C gerührt, mit Wasser bei 0°C versetzt und mehrmals mit Diethylether extrahiert. Die vereinigten organischen Phasen wurden mit Wasser und ges. Natriumchloridlösung gewaschen, über Magnesiumsulfat trocknet und eingeengt. Säulenchromatographische Reinigung (Cyclohexan/Essigester 3:1) ergab 67 mg **23** als farbloser Feststoff (Fp: 142-145°C).

### 11β-Hexyl-8β-vinyl-estra-1,3,5(10)-trien-3,17β-diol (25)

Zu einer Lösung von 65 mg 3-Methylether **23** in 3.3 ml abs. Toluol wurden bei 0°C 0.37 ml DIBAH getropft. Anschließend wurde für 4 Stunden unter Rückfluss erhitzt. Nach Abkühlen der Reaktionslösung auf 0°C wurde diese nacheinander mit 1.6 ml Ethanol, 1.6 ml Ethanol/Wasser (1:1) und 1.6 ml halbkonz. Salzsäure versetzt, anschließend mehrmals mit Essigester extrahiert, die vereinigten organischen Phasen mit Wasser und ges. Natriumchloridlösung gewaschen, über Magnesiumsulfat getrocknet und eingeengt. Säulenchromatographie (Cyclohexan/Essigester 2:1) des Rückstandes ergab 50 mg **25** als farblosen Feststoff (Fp: 148-150°C).

## Patentansprüche

1. 8β-substituierte Estra-1,3,5(10)-trienderivate der allgemeinen Formel I 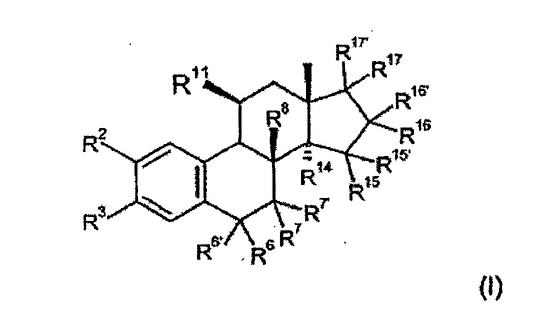 worin
R²: Wasserstoff, F, Cl, Br, I;
einen Rest R¹⁸ oder R¹⁸O, wobei R¹⁸ Wasserstoff, ein Alkyl- oder Acylrest beide gerad- oder verzweigtkettig, gesättigt oder ungesättigt mit bis zu 6 Kohlenstoffatomen, eine Trifluormethylgruppe;
einen Rest R¹⁹SO₂O, wobei R¹⁹ eine R²⁰R²¹N-Gruppe, worin R²⁰ und R²¹ unabhängig voneinander ein Wasserstoff, einen C₁-C₅- Alkylrest, eine Gruppe C(O)R²², worin R²² einen Kohlenwasserstoffrest gegebenenfalls substituiert, gerad- oder verzweigtkettig, gesättigt oder bis zu dreifach ungesättigt, teilweise oder vollständig halogeniert mit bis zu 10 Kohlenstoffatomen, einen gegebenenfalls substituierten C₃-C₇ Cycloalkylrest, einen gegebenenfalls substituierten C₄-C₁₅-Cycloalkylalkylrest oder einen gegebenenfalls substituierten Aryl-, Heteroaryl- oder Aralkylrest, oder, zusammen mit dem N-Atom, einen Polymethyleniminorest mit 4 bis 6 C-Atomen oder einen Morpholinorest;
R³: R¹⁸O, R¹⁹SO₂O, OC(O)R²², mit R¹⁸, R¹⁹, R²² in der unter R² angegebenen Bedeutung, sowie R¹⁸ zusätzlich einen Aryl-, Hetaryl- oder Aralkyl-Rest;
R⁶, R^{6'}: je ein Wasserstoff oder R⁶ eine zusätzliche Bindung mit R⁷;
R⁷, R^{7'}: je ein Wasserstoff oder R⁷ eine zusätzliche Bindung mit R⁶;
R⁸: einen Alkyl-, Alkenylrest gerad- oder verzweigtkettig, teilweise oder vollständig halogeniert, jeweils mit bis zu 5 Kohlenstoffatomen, einen Ethinyl oder Prop-1-inylrest;
R¹¹: einen n-Pentyl- oder n-Hexylrest
R¹⁴: Wasserstoff oder eine zusätzliche Bindung mit R¹⁵;
R¹⁵: Wasserstoff oder eine zusätzliche Bindung mit R¹⁴ oder R¹⁶;
R¹⁶: Wasserstoff oder eine zusätzliche Bindung mit R¹⁵
R^{15'}, R^{16'}: unabhängig voneinander Wasserstoff, Halogen, eine Gruppe R¹⁸O, R¹⁹SO₂O oder OC(O)R²² mit R¹⁸, R¹⁹, R²² in der unter R² angegebenen Bedeutung;
R¹⁷, R^{17'}: je ein Wasserstoffatom;
ein Wasserstoffatom und ein Halogenatom;
ein Wasserstoffatom und eine Benzyloxygruppe;
ein Wasserstoffatom und eine Gruppe R¹⁹SO₂-O-;
eine Gruppe R¹⁸ und eine Gruppe -C(O)R²² oder -O-C(O)R²²;
eine Gruppe R¹⁸-O- und eine Gruppe R¹⁸-;
eine Gruppe R¹⁸-O- und eine Gruppe -O-C(O)R²², mit R¹⁸, R¹⁹ und R²² in der unter R² angegebenen Bedeutung oder
R¹⁷, R^{17'}: gemeinsam eine Gruppe =CR²³R²⁴, worin R²³ und R²⁴ unabhängig voneinander ein Wasserstoffatom und ein Halogenatom, oder gemeinsam ein Sauerstoffatom darstellen;
bedeuten, worin: Alkylgruppen bzw Kohlenstoffreste teilweise oder vollständig fluoriert oder substituiert durch 1-5 Halogenatome, Hydroxygruppen oder C₁₋₄-Alkoxygruppen sein können; ein C₄₋₁₅-Cycloalkylalkylrest 3 bis 7 Kohlenstoffatome im Cycloalkylteil und bis zu 8 Kohlenstoffatome im Alkylteil aufweist; Aryl Phenyl-, 1- oder 2-Naphthyl bedeutet; Aralkyl im Ring bis zum 14 C-Atome und in der Alkylkette 1 bis 8 C-Atome aufweist; ein oder mehrere Hydroxylgruppen an den C-Atomen 3, 16 und 17 mit einer aliphatischen, gerad- oder verzweigtkettigen, gesättigten oder ungesättigten C₁₋₁₄-Mono- oder Poly-carbonsäure oder einer aromatischen Carbonsäure oder mit einer α- oder β-Aminsäure verestert sein können.

2. Verbindungen der allgemeinen Formel I nach Anspruch 1, nämlich:
8β-Methyl-11β-pentyl-estra-1,3,5(10)-trien-3,17β-diol
8β-Ethyl-11β-pentyl-estra-1,3,5(10)-trien-3,17β-diol
11β-Pentyl-8β-vinyl-estra-1,3,5(10)-trien-3,17β-diol
11β-Hexyl-8β-methyl-estra-1,3,5(10)-trien-3,17β-diol
8β-Ethyl-11β-hexyl-estra-1,3,5(10)-trien-3,17β-diol
11β-Hexyl-8β-vinyl-estra-1,3,5(10)-trien-3,17β-diol
8β-Methyl-11β-pentyl-estra-1,3,5(10)-trien-3,17β-diol-3-sulfamat
8β-Ethyl-11β-pentyl-estra-1,3,5(10)-trien-3,17β-diol-3-sulfamat
11β-Pentyl-8β-vinyl-estra-1,3,5(10)-trien-3,17β-diol-3-sulfamat
11β-Hexyl-8β-methyl-estra-1,3,5(10)-trien-3,17β-diol-3-sulfamat
8β-Ethyl-11β-hexyl-estra-1,3,5(10)-trien-3,17β-diol-3-sulfamat
11β-Hexyl-8β-vinyl-estra-1,3,5(10)-trien-3,17β-diol-3-sulfamat
8β-Methyl-11β-pentyl-estra-1,3,5(10)-trien-3,17β-diol-3-acetat
8β-Ethyl-11β-pentyl-estra-1,3,5(10)-trien-3,17β-diol-3-acetat
11β-Pentyl-8β-vinyl-estra-1,3,5(10)-trien-3,17β-diol-3-acetat
11β-Hexyl-8β-methyl-estra-1,3,5(10)-trien-3,17β-diol-3-acetat
8β-Ethyl-11β-hexyl-estra-1,3,5(10)-trien-3,17β-diol-3-acetat
11β-Hexyl-8β-vinyl-estra-1,3,5(10)-trien-3,17β-diol-3-acetat.

3. Verwendung der 8β-substituierten Estra-1,3,5( 10)-trienderivate der allgemeinen Formel I gemäß Anspruch 1 zur Herstellung von Arzneimitteln für die Kontrazeption bei der Frau.

4. Verwendung der 8β-substituierten Estra-1,3,5( 10)-trienderivate der allgemeinen Formel I gemäß Anspruch 1 zur Herstellung von Arzneimitteln für die Kontrazeption beim Mann.

5. Verwendung der Estratrienderivate der allgemeinen Formel I gemäß Anspruch 1 zur Herstellung von Arzneimitteln zur Behandlung von gutartigen oder bösartigen proliferativen Erkrankungen des Ovars.

6. Verwendung nach Anspruch 5 zur Herstellung von Arzneimitteln zur Behandlung von Ovarialcarcinomen.

7. Verwendung nach Anspruch 5 zur Herstellung von Arzneimitteln zur Behandlung von Granulosazelltumoren.

8. Pharmazeutische Zusammensetzungen, enthaltend mindestens eine Verbindung gemäß einem der Ansprüche 1 bis 3 sowie einen pharmazeutisch verträglichen Träger.

9. Pharmazeutische Zusammensetzungen nach Anspruch 8, die neben mindestens einer Verbindung der allgemeinen Formel I gemäß Anspruch 1 mindestens eine Verbindung ausgewählt aus der Gruppe der GnRH-Antagonisten, Progesteronrezeptorantagonisten, Mesoprogestinen, Gestagenen oder gewebeselektiven Gestagenen enthalten.

## Claims

1. 8β-Substituted estxa-1,3,5(10)-triene derivatives of general formula I 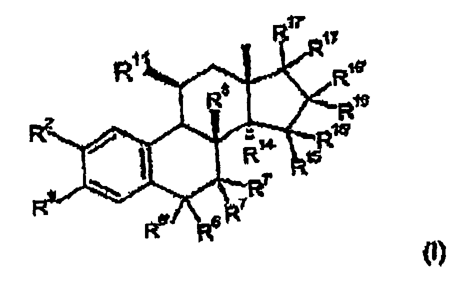 in which
R²: means hydrogen, F, Cl, Br, I,;
a radical R¹⁸ or R¹⁸O, where R¹⁸ means hydrogen, an alkyl or acyl radical, both straight-chain or branched-chain, saturated or unsaturated with up to 6 carbon atoms, a trifluoromethyl group;
a radical R¹⁹SO₂O, where R¹⁹ means an R²⁰R²¹N group, in which R²⁰ and R²¹, independently of one another, mean hydrogen, a C₁-C₅-alkyl radical, a group C(O)R²², in which R²² means a hydrocarbon radical, optionally substituted, straight-chain or branched-chain, saturated or unsaturated in up to three places, partially or completely halogenated, with up to 10 carbon atoms, an optionally substituted C₃-C₇-cycloalkyl radical, an optionally substituted C₄-C₁₅-cycloalkylalkyl radical or an optionally substituted aryl, heteroaryl or aralkyl radical, or, together with the N-atom, means a polymethylenimino radical with 4 to 6 C atoms or a morpholino radical;
R³: means R¹⁸O, R¹⁹SO₂O or OC(O)R²², with R¹⁸, R¹⁹ and R²² in the meaning that is indicated under R², and in addition R¹⁸ means an aryl, hetaryl or aralkyl radical;
R⁶, R^{6'}: each mean hydrogen or R⁶ means an additional bond with R⁷;
R⁷, R^{7'}: each mean hydrogen, or R⁷ means an additional bond with R⁶;
R⁸: means an alkyl or alkenyl radical, straight-chain or branched-chain, partially or completely halogenated, in each case with up to 5 carbon atoms, an ethynyl or prop-1-ynyl radical;
R¹¹: means an n-pentyl or n-hexyl radical;
R¹⁴: means hydrogen or an additional bond with R¹⁵; R¹⁵: means hydrogen or an additional bond with R¹⁴ or R¹⁶;
R¹⁶: means hydrogen or an additional bond with R¹⁵; R^{15'}, R^{16'}: independently of one another, mean hydrogen, halogen, a group R¹⁸O, R¹⁹SO₂O or OC(O)R²², with R¹⁸, R¹⁹ and R²² in the meaning that is indicated under R²;
R¹⁷, R^{17'} : each mean a hydrogen atom;
a hydrogen atom and a halogen atom;
a hydrogen atom and a benzyloxy group;
a hydrogen atom and a group R¹⁹SO₂-O-;
a group R¹⁸ and a group -C(O)R²² or -O-C(O)R²²;
a group R¹⁸-O- and a group R¹⁸-;
a group R¹⁸-O- and a group -O-C(O)R²², with R¹⁸, R¹⁹ and R²² in the meaning that is indicated under R²; or
R¹⁷, R^{17'}: together mean a group =CR²³R²⁴, in which R²³ and R²⁴, independently of one another, represent a hydrogen atom and a halogen atom, or together represent an oxygen atom
in which: alkyl groups and carbon radicals can be partially or completely fluorinated or substituted by 1-5 halogen atoms, hydroxyl groups or C₁₋₄-alkoxy groups; a C₄₋₁₅-cyclo-alkylalkyl radical has 3 to 7 carbon atoms in the cycloalkyl portion and up to 8 carbon atoms in the alkyl portion; aryl means phenyl, 1- or 2-naphthyl; aralkyl contains in the ring up to 14 C atoms and in the alkyl chain 1 to 8 C atoms; one or more hydroxyl groups at C atoms 3, 16 and 17 can be esterified with an aliphatic, straight-chain or branched-chain, saturated or unsaturated C₁₋₁₄-mono- or polycarboxylic acid or an aromatic carboxylic acid or with an α- or β-amino acid.

2. Compounds of general formula I according to Claim 1, namely
8β-Methyl-11β-pentyl-estra-1,3,5(10)-triene-3,17β-diol
8β-ethyl-11β-pentyl-estra-1,3,5(10)-triene-3,17β-diol
11β-pentyl-8β-vinyl-estra-1,3,5(10)-triene-3,17β-diol
11β-hexyl-8β-methyl-estra-1,3,5(10)-triene-3,17β-diol
8β-ethyl-11β-hexyl-estra-1,3,5(10)-triene-3,17β-diol
11β-hexyl-8β-vinyl-estra-1,3,5(10)-triene-3,17β-diol
8β-methyl-11β-pentyl-estra-1,3,5(10)-triene-3,17β-diol-3-sulfamate
8β-ethyl-11β-pentyl-estra-1,3,5(10)-triene-3,17β-diol-3-sulfamate
11β-pentyl-8β-vinyl-estra-1,3,5(10)-triene-3,17β-diol-3-sulfamate
11β-hexyl-8β-methyl-estra-1,3,5(10)-triene-3,17β-diol-3-sulfamate
8β-ethyl-11β-hexyl-estra-1,3,5(10)-triene-3,17β-diol-3-sulfamate
11β-hexyl-8β-vinyl-estra-1,3,5(10)-triene-3,17β-diol-3-sulfamate
8β-methyl-11β-pentyl-estra-1,3,5(10)-triene-3,17β-diol-3-acetate
8β-ethyl-11β-pentyl-1,3,5(10)-triene-3,17β-diol-3-acetate
11β-pentyl-8β-vinyl-estra-1,3,5(10)-triene-3,17β-diol-3-acetate
11β-hexyl-8β-methyl-estra-1,3,5(10)-triene-3,17β-diol-3-acetate
8β-ethyl-11β-hexyl-estra-1,3,5(10)-triene-3,17β-diol-3-acetate
11β-hexyl-8β-vinyl-estra-1,3,5(10)-triene-3,17β-diol-3-acetate.

3. Use of 8β-substituted estra-1,3,5(10)-triene derivatives of general formula I according to Claim 1 for the production of pharmaceutical agents for contraception in women.

4. Use of 8β-substituted estra-1,3,5(10)-triene derivatives of general formula I according to Claim 1 for the production of pharmaceutical agents for contraception in men.

5. Use of the estratriene derivatives of general formula I according to Claim 1 for the production of pharmaceutical agents for treating benign or malignant proliferative diseases of the ovary.

6. Use according to Claim 5 for the production of pharmaceutical agents for treating ovarian cancer.

7. Use according to Claim 5 for the production of pharmaceutical agents for treating granulosa cell tumours.

8. Pharmaceutical compositions that contain at least one compound according to one of Claims 1 to 3, as well as a pharmaceutically compatible vehicle.

9. Pharmaceutical compositions according to Claim 8, which in addition to at least one compound of general formula I according to Claim 1 contain at least one compound that is selected from the group of GnRH antagonists, progesterone receptor antagonists, mesoprogestins, gestagens or tissue-selective gestagens.

## Revendications

1. Dérivés d'estra-1,3,5(10)-triène substituée en position 8β de formule générale I : 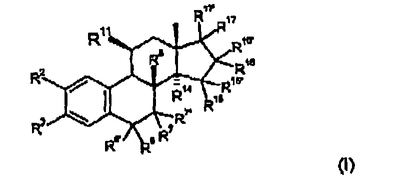 dans laquelle
R² : représente un hydrogène, F, Cl, Br, I ; un radical R¹⁸ ou R¹⁸O, dans lequel R¹⁸ représente un hydrogène, un radical alkyle ou un radical acyle, tous les deux linéaires ou ramifiés, saturés ou insaturés, renfermant jusqu'à 6 atomes de carbone, un groupe trifluorométhyle ;
un radical R¹⁹SO₂O, dans lequel R¹⁹ représente un groupe R²⁰R²¹N, dans lequel R²⁰ et R²¹ représentent, indépendamment l'un de l'autre, un hydrogène, un radical alkyle en C₁-C₅, un groupe C(O)R²², dans lequel R²² représente un radical hydrocarboné éventuellement substitué, linéaire ou ramifié, saturé ou jusqu'à tri-insaturé, partiellement ou totalement halogéné avec jusqu'à 10 atomes de carbone, un radical cycloalkyle en C₃-C₇, éventuellement substitué, un radical cycloalkylalkyle en C₄-C₁₅, éventuellement substitué ou un radical aryle, hétéroaryle ou aralkyle éventuellement substitué, ou, conjointement avec l'atome d'azote, un radical polyméthylène-imino ayant de 4 à 6 atomes de carbone ou un radical morpholino ;
R³ : représente R¹⁸O, R¹⁹SO₂₀, OC(O)R²², dans lesquels R¹⁸, R¹⁹ et R²² sont tels que définis pour R², et R¹⁸ représente en outre un radical aryle, hétaryle ou aralkyle ;
R⁶, R^{6'} : représentent chacun un hydrogène ou R⁶ représente une liaison supplémentaire avec R⁷ ;
R⁷, R^{7'} : représentent chacun un hydrogène ou R⁷ représente une liaison supplémentaire avec R⁶ ;
R⁸ : représente un radical alkyle, un radical alcényle, linéaire ou ramifié, partiellement ou totalement halogéné, dans chaque cas ayant jusgu'à 5 atomes de carbone, un radical éthynyle ou Prop-1-inyle ;
R¹¹ : représente un radical n-pentyle ou n-hexyle ;
R¹⁴ : représente un hydrogène ou une liaison supplémentaire avec R¹⁵ ;
R¹⁵ : représente un hydrogène ou une liaison supplémentaire avec R¹⁴ ou R¹⁶ ;
R¹⁶ : représente un hydrogène ou une liaison supplémentaire avec R¹⁵ ;
R^{15'}, R^{16'} : représentent, indépendamment l'un de l'autre, un hydrogène, un halogène, un groupe R¹⁸O, R¹⁹SO₂O, ou OC(O)R²², dans lequel R¹⁸, R¹⁹, R²² sont tels que définis pour R² ;
R¹⁷, R^{17'} : représentent chacun un atome d'hydrogène ;
un atome d'hydrogène et un atome d'halogène ;
un atome d'hydrogène et un groupe benzyloxy ;
un atome d'hydrogène et un groupe R¹⁹SO₂O, ;
un groupe R¹⁸ et un groupe -C(O)R²² ou -OC(O)R²² ;
un groupe R¹⁸ -0- et un groupe R¹⁸- ;
un groupe R¹⁸-O- et un groupe OC(O)R²², dans lesquels R¹⁸, R¹⁹ et R²², sont tels que définis pour R², ou
R¹⁷ R^{17'} : représentent conjointement un groupe =CR²³R²⁴, dans lequel R²³ et R²⁴ représentent indépendamment l'un de l'autre un atome d'hydrogène et un atome d'halogène, ou représentent conjointement un atome d'oxygène :
où :
les groupes alkyle ou les radicaux carbonés peuvent être partiellement ou totalement fluorés ou substitués par de 1 à 5 atomes d'halogène, groupes hydroxy ou groupes alcoxy en C₁-C₄; un radical cycloalkylalkyle en C₄-C₁₅ renferme de 3 à 7 atomes de carbone dans la portion cycloalkyle et jusqu'à 8 atomes de carbone dans la portion alkyle ; un aryle représente un phényle, un 1- ou 2-naphtyle ; un aralkyle renferme jusqu'à 14 atomes de carbone dans le noyau et de 1 à 8 atomes de carbone dans la chaîne alkyle ; un ou plusieurs groupes hydroxyle peuvent être estérifiés sur les atomes de carbone 3, 16 et 17 avec un acide C₁₋₁₄-mono ou polycarboxylique aliphatique, linéaire ou ramifié, saturé ou insaturé ou un acide carboxylique aromatique, ou avec un acide α- ou β-amino.

2. Composés de formule I selon la revendication 1, à savoir :
le 8β-méthyl-11β-pentylestra-1,3,5(10)-triène-3,17β-diol
le 8β-éthyl-11β-pentylestra-1,3,5(10)-triène-3,17β-diol
le 11β-pentyl-8β-vinylestra-1,3,5(10)-triène-3,17β-diol
le 11β-hexyl-8β-méthylestra-1,3,5(10)-triène-3,17β-diol
le 8β-éthyl-11β-hexylestra-1,3,5(10)-triène-3,17β-diol
le 11β-hexyl-8β-vinylestra-1,3,5(10)-triène-3,17β-diol
le 8β-méthyl-11β-pentylestra-1,3,5(10)-triène-3,17β-diol-3-sulfamate
le 8β-éthyl-11β-pentylestra-1,3,5(10)-triène-3,17β-diol-3-sulfamate
le 11β-pentyl-8β-vinylestra-1,3,5(10)-triène-3,17β-diol-3-sulfamate
le 11β-hexyl-8β-méthylestra-1,3,5(10)-triène-3,17β-diol-3-sulfamate
le 8β-éthyl-11β-hexylestra-1,3,5(10)-triène-3,17β-diol-3-sulfamate
le 11β-hexyl-8β-vinylestra-1,3,5(10)-triène-3,17β-diol-3-sulfamate
le 8β-méthyl-11β-pentylestra-1,3,5(10)-triène-3,17β-diol-3-acétate
le 8β-éthyl-11β-pentylestra-1,3,5(10)-triène-3,17β-diol-3-acétate
le 11β-pentyl-8β-vinylestra-1,3,5(10)-triène-3,17β-diol-3-acétate
le 11β-hexyl-8β-méthylestra-1,3,5(10)-triène-3,17β-diol-3-acétate
le 8β-éthyl-11β-hexylestra-1,3,5(10)-triène-3,17β-diol-3-acétate
le 11β-hexyl-8β-vinylestra-1,3,5(10)-triène-3,17β-diol-3-acétate

3. Utilisation des dérivés d'estra-1,3,5(10)-triène substitué en position 8β de formule générale I selon la revendication 1 pour la préparation d'agents pharmaceutiques destinés à la contraception chez la femme.

4. Utilisation des dérivés d'estra-1,3,5(10)-triène substitués en position 8β de formule générale I selon la revendication 1 pour la préparation d'agents pharmaceutiques destinés à la contraception chez l'homme.

5. Utilisation des dérivés d'estratriène de formule générale I selon la revendication 1 pour la préparation d'agents pharmaceutiques destinés au traitement de troubles prolifératifs bénins ou malins de l'ovaire.

6. Utilisation selon la revendication 5 pour la préparation d'agents pharmaceutiques destinés au traitement de carcinomes ovariens.

7. Utilisation selon la revendication 5 pour la préparation d'agents pharmaceutiques destinés au traitement de tumeurs à cellules de la granulosa.

8. Compositions pharmaceutiques comprenant au moins un composé selon l'une quelconque des revendications 1 à 3 ainsi qu'un support pharmaceutiquement acceptable.

9. Compositions pharmaceutique selon la revendication 8, comprenant en plus d'au moins un composé de formule générale I selon la revendication 1, au moins un composé choisi parmi le groupe constitué d'antagonistes de GnRH, d'antagonistes de récepteur de la progestérone, de mésoprogestatifs, de progestatifs ou de progestatifs sélectifs vis-à-vis du tissu.
